# EUROPEAN PATENT SPECIFICATION

(11) **EP 0 570 560 B1**
(45) Date of publication and mention of the grant of the patent: **28.10.1998**
(21) Application number: 92924685.8
(22) Date of filing: 07.12.1992
(51) Int. Cl.: C07D 213/22, C07D 213/38, G01N 24/00

(54) **ION-SENSITIVE COMPOUNDS**
ION-EMPFINDLICHE VERBINDUNGEN
COMPOSES A SENSIBILITE IONIQUE

(30) Priority: 10.12.1991 GB 9126146
(43) Date of publication of application: 24.11.1993
(73) Proprietor: KODAK LIMITED, Harrow, Middlesex HA1 4TY (GB); EASTMAN KODAK COMPANY, Rochester, New York 14650-2201 (US)
(72) Inventor: WEAR, Trevor John, South Harrow, Middlesex HA2 8TD (GB); MOORE, Christopher Peter, Harrow, Middlesex HA2 9QD (GB); BEER, Paul, D., Oxford OX2 8DF (GB); WHEELER, John W., Moseley, Birmingham B13 9PA (GB)
(74) Representative: Nunney, Ronald Frederick Adolphe
(86) International application number: EP9202830
(87) International publication number: WO9312088

(56) References cited:
- JOURNAL OF THE CHEMICAL SOCIETY, CHEMICAL COMMUNICATIONS no. 17, 1 September 1992, LETCHWORTH GB pages 1225 - 1227 P.D. BEER ET AL. 'Anion recognition by new acyclic quaternary polybipyridinium receptors'
- CHEMICAL ABSTRACTS, vol. 108, no. 3, 18 January 1988, Columbus, Ohio, US; abstract no. 21736h, P.G. POTVIN ET AL. 'Design of cation and anion receptors, catalysts, and carriers.' page 586 ; & PROGRESS IN MACROCYCLIC CHEMISTRY, no. 3, 1987, New York, pages 167 - 239
- BULLETIN DE LA SOCIETE CHIMIQUE DE FRANCE no. 11, 1983, PARIS FR pages 367 - 380 J.-L. PIERRE ET AL. 'Complexes moléculaires d'anions'

## Description

The invention relates to ion-sensitive compounds. More particularly, the invention relates to ion-sensitive compounds comprising a quaternary polypyridinium receptor designed to bind anionic species by the formation of a receptor-substrate complex.

Anion receptors comprising a plurality of quaternary amine groups are known. Examples of such compounds may be seen in Chemical Abstracts, vol. 108, no. 3, 18 January 1988, Columbus, Ohio, US; abstract no. 21736h, P.G. Potvin et al. "Design of cation and anion receptors, catalysts and carriers.", page 586 & Progress in Macrocyclic Chemistry, no. 3, 1987, pages 167-239, especially page 214.

L.A. Summers, "The Bipyridinium Herbicides", Academic Press, New York, 1980, describes the use of certain compounds comprising diquaternary 2,2'-bipyridinium moieties in herbicidal applications.

There is a continuing need to provide new receptor compounds for a variety of applications. For example, such compounds may be incorporated in electrochemical sensors for anion determination. A number of electrochemical sensors utilising ion receptors are known. Alternatively, such compounds may be used in removal devices where levels of a given anion need to be kept low. A number of removal devices utilising ion receptors are known. It is also desirable to provide receptor compounds which can be readily synthesised.

The invention provides a new class of anion receptors.

The ion-sensitive compounds of the invention have the formula Aⁿ⁺Bⁿ⁻ wherein A represents a cation capable of forming a receptor-substrate complex with an anion, B represents one or more counter anions and n is an integer from 3 to 10, characterised in that the cation is an anion receptor represented by the formula R¹-Y-R² wherein Y is represented by the structure 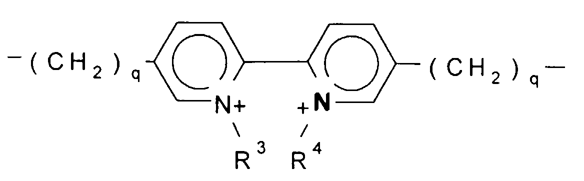
each q independently is an integer from 1 to 6;
R³ and R⁴ are each independently H or a lower alkyl group having from 1 to 4 carbon atoms, or R³ and R⁴ taken together represent an ethylene bridging group;
R¹ and R² are each independently selected from organic cations and non-ionic organic groups wherein at least one of R¹ and R² is an organic cation and the combined ionic charge of R¹ and R² is from 1 to 8 wherein
   (a) the organic cation is selected from pyridinium, bipyridinium, alkylammonium, poly(alkylammonium), arylammonium and poly(arylammonium);
   (b) the non-ionic organic group is selected from alkylamino, poly(alkylamino), arylamino, poly(arylamino), alkylamido, arylamido, alkylphosphoramido, arylphosphoramido, alkylsulphonamido, arylsulphonamido, alkyloxycarbonyl, aryloxycarbonyl, pyridyl and bipyridyl; or,
R¹ is H and R² is -X-Y-H wherein X is selected from alkylene, arylene, and quaternised or unquaternised amine- or polyamine-containing groups and Y is as defined above; or,
R¹ and R² taken together represent -X-Y-X-wherein X and Y are as defined above.

Preferred compounds having the formula defined above include compounds wherein q is 1. R³ and R⁴ may each be methyl.

Examples of organic cations from which R¹ and R² may be chosen include the quaternised forms of the non-ionic groups listed above. Particular examples are 2,2'-bipyridinium, 4,4'-bipyridinium and -[⁺N(R⁵)₂-R⁶]ₚ-R⁷ wherein each R⁵ independently is hydrogen or an alkyl group of from 1 to 4 carbon atoms; R⁶ is an alkylene group of from 1 to 3 carbon atoms; R⁷ is hydrogen or -N(R⁵)₃ and p is an integer from 1 to 4.

Preferred compounds include those wherein both R¹ and R² are organic cations.

Examples of suitable linking groups represented by X include alkylene, arylene, and quaternised or unquaternised amine- or polyamine-containing groups such as alkylamino, arylamino, aminoalkyleneamino, amino[poly (alkyleneamino)], bipyridyl and bipyridylamino. Preferred groups include 4,4'-bipyridinium and -[⁺N(R⁵)₂-R⁶]ₚ-N⁺(R⁵)₂- wherein R⁵, R⁶ and p are as defined above.

In the definitions given above, all alkyl, alkylene, aryl and arylene groups, when present as such or as part of another substituent, are optionally substituted.

Specific examples of preferred compounds of the invention are as follows.

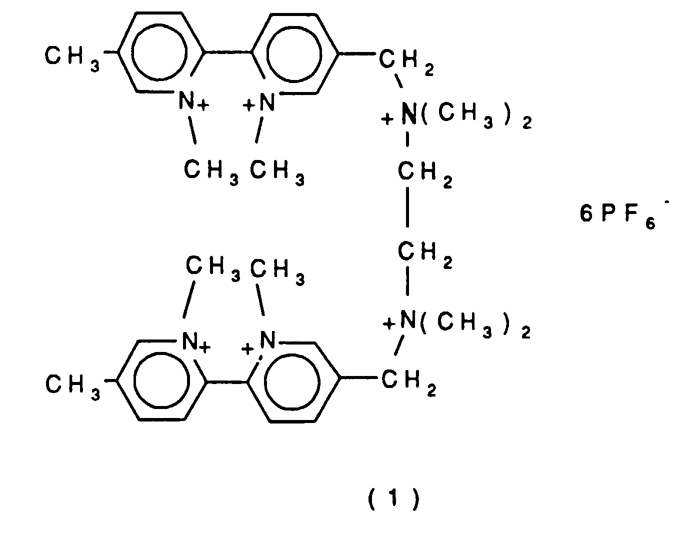

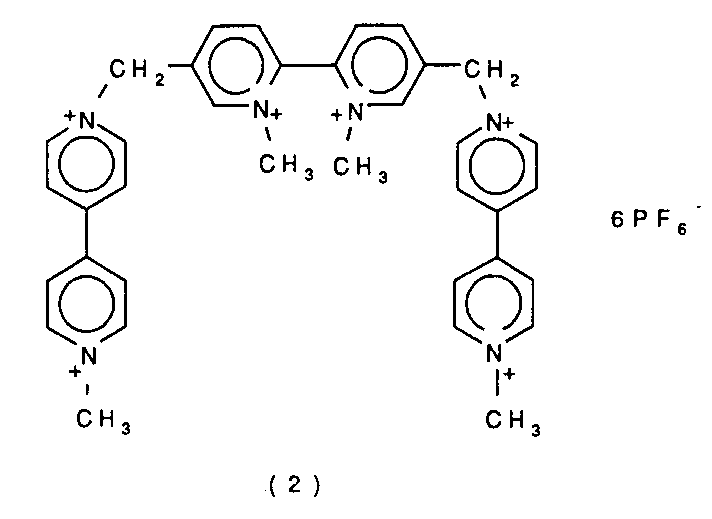

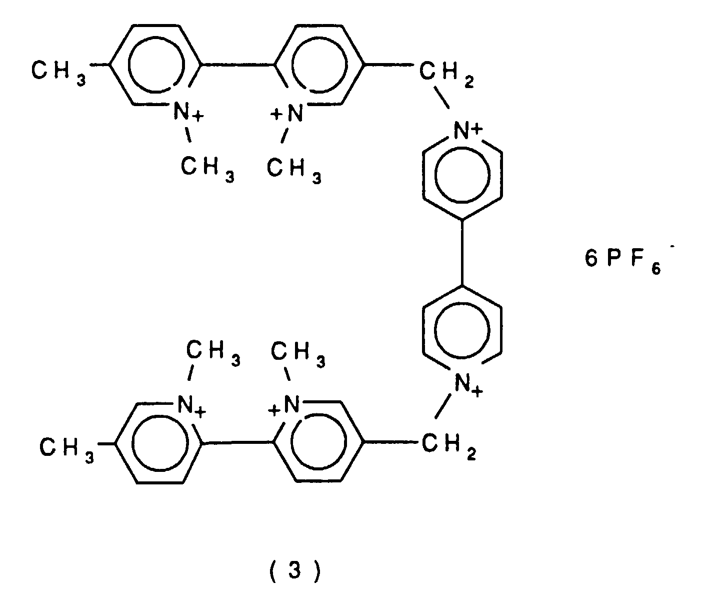

The compounds of the invention can be prepared using 5,5'-dibromoalkyl-2,2'-bipyridyl or 5-bromoalkyl-5'-alkyl-2,2'-bipyridyl as the starting materials. Reaction of the dibromo substituted material with an appropriate amine e.g. 4,4'-bipyridyl followed by quaternisation with an alkylating agent e.g. methyl iodide will produce the 5,5'-disubstituted compounds of the invention in which the 2,2'-bipyridinium moiety is flanked by positively charged groups. Alternatively, the dibromo compound can be reacted with a variey of compounds which have been deprotonated to give a mono-anion e.g. the mono anion produced by deprotonating 2,2'-bipyridyl with butyl lithium.

Reaction of the monobromo substituted material with an appropriate diamine e.g. N,N'-dimethyl ethylenediamine followed by quaternisation with an alkylating agent will produce compounds of the invention in which two 2,2'-bipyridinium moieties are attached through a linking group. Other bifunctional linking groups may be reacted with the monobromo substituted material in this way with or without formation of a di-anion by deprotonation.

Specific examples of the preparation of compounds of the invention are given as follows.

### Example 1

### N,N'-Bis(5'-methyl-2,2'-bipyridyl-5-methylene)-N,N'-dimethyl-ethylene-diamine

N,N'-Dimethyl ethylene diamine (76mg. 0.86mmol) was dissolved in acetonitrile (30cm³), potassium carbonate (2.64g, 19.1mmol) added and the mixture heated to reflux under nitrogen. To this was added dropwise a solution of 5-bromomethyl-5'-methyl-2,2'-bipyridyl (0.50g, 1.90mmol) in acetonitrile (30cm³) with stirring and the resultant mixture heated at reflux for 3h. After this time the mixture was cooled to room temperature, the solid removed by filtration and the solvent removed from the filtrate under reduced pressure to give a deep orange residue. This was then chromatographed on alumina using dichloromethane/methanol (99.1) as elutant. A pale orange band was collected. After removal of the solvent the product was recrystallised from acetonitrile to give small off-white crystals of the required ligand (0.17g, 43%) m.p. 160-161°C.
M.S.(FAB) [m/z+H]⁺ 453,[CH₂bipyCH₂N(Me)CH₂]⁺ 226, [CH₂bipyCH₂]⁺ 183.
I.R. 3000cm⁻¹ (aromatic CH stretch), 2850/2800cm⁻¹ (aliphatic CH stretch), 1600/1580cm⁻¹ (C=C/C=N ring stretch)
¹H NMR (CDCl₃, 270MHz) δ: 2.24 (6H, s, bipyCH₂NCH₃), 2.39 (6H, s, bipyCH₃), 2.59 (4H, s, bipyCH₂NCH₂), 3.58 (4H, s, bipyCH₂), 7.61 (2H, d, ³J=8.1Hz, bipyH_{4'}), 7.77 (2H, d, ³J=8.2Hz, bipyH₄), 8.26 (2H, d, ³J=8.2Hz, bipyH₃), 8.30 (2H, d, ³J=8.1Hz,bipyH_{3'}), 8.49 (2H, s, bipyH_{6'}), 8.56 (2H, s, bipyH₆)
¹³C NMR (CDCl₃, 67.8MHz) δ: 18.35 (bipyCH₃). 42.55 (bipyCH₂NCH₃), 55.11 and 59.76 (bipyCH₂NCH₂), 120.41, 120.50, 133.22, 134.12, 137.43, 137.57, 149.59, 149.70, 153.59 and 155.25 (aromatic C)
Analysis calculated for C₂₈H₃₂N₆; C:74.3%, H:7.1%, N:18.6%. Found C:74.5%, H:6.8%, N:18.9%.

### N,N'-Bis(N,N',5'-trimethyl-2,2'-bipyridyl-5-methylene)-N,N,N',N'-tetramethyl-ethylene-diammonium hexa (hexafluorophosphate) (1)

The ligand (70mg, 0.16mmol) was dissolved in dimethyl sulphate (10cm³) with stirring and heated to 75°C under nitrogen for 7 days. The solution was cooled to room temperature and the precipitate collected for filtration. The solid was washed with acetone (3 x 10cm³) and then dissolved in water (15cm³). To this solution was added a saturated aqueous solution of ammonium hexafluorophosphate until precipitation ceased to occur. The precipitate was collected by filtration, washed with water (2 x 10cm³) and dried under vacuum over silica gel to give a white solid (73mg).

The solid was dissolved in acetonitrile under nitrogen with stirring. To this was added methyl iodide and the resultant solution heated at reflux for 18 days. After this time the mixture was cooled to room temperature. A solution of tetra-butyl ammonium chloride (0.2g in 5cm³ of acetonitrile) was added to precipitate the product as the chloride salt. This was collected by filtration, dried, then dissolved in water (10cm³). To the solution was added a saturated aqueous solution of ammonium hexafluorophosphate until precipitation ceased to occur. The solid was collected by filtration, washed with water (2 x 5cm³) and dried under vacuum over silica to give the hexacationic receptor (1) (24mg, 11%) m.p. 195°C (decomp). M.S.(FAB) [m/z-PF₆-]⁺ 1267, [m/z-2PF₆-]⁺ 1122, [m/z- CH₃⁺-2PF₆-]⁺ 1107, [m/z-CH₃⁺-3PF₆-]⁺ 962, [m/z-4CH₃+-5PF₆-]⁺ 629
¹H NMR (DMSO, 400MHz) δ: 2.68 (6H, s, bipyCH₃), 3.21 and 3.24 (12H, 2s, bipyCH₂N⁺(CH₃)₂, 4.14 and 4.24 (12H, 2s, bipyN⁺-CH₃), 4.27 (4H, s, bipyCH₂N⁺CH₂) 5.02 (4H, s, bipyCH₂), 8.19 (2H, d, ³J=8.1Hz, bipyH_{4'}), 8.63 (2H, d, ³J=8.1Hz, bipyH₄), 8.79 (2H, d, ³J=8.1Hz), bipyH_{3'}), 9.07 (2H, d, ³J=8.1Hz, bipyH₃), 9.42 (2H, s, bipyH_{6'}), 9.53 (2H, s, bipyH₆)
¹³C NMR (DMSO, δ: 18.12 (bipyCH₃), 47.42 and 48.11 (bipyN⁺-CH₃), 49.58 and 49.74 (bipyCH₂N⁺(CH₃)₂), 57.98 (bipyCH₂N⁺CH₂), 62.60 (bipyCH₂), 129.47, 129.70, 131.22, 139.78, 141.63, 144.67, 146.72, 149.14, 151.00 and 152.09 (aromatic C).
Analysis calculated for C₃₄H₅₀F₃₆N₆P₆; C:28.9%, H:3.6%, N:6.0%. Found C:29.4%, H:3.6%, N:6.1%

### Anion Binding

The receptor (1) displayed substantial shifts of the signals of the ¹H NMR spectrum following the addition of one equivalent of tetrabutyl ammonium chloride. Analysis of the signals confirmed the formation of a complex between the receptor and the chloride ion.

### Example 2

### 5,5'-Bis(4,4'-bipyridyl-N-methylene)-2,2'-bipyridyl bis(hexafluorophosphate)

4,4'-Bipyridyl (18.2g, 116.5mmol) was dissolved in acetonitrile (700cm³) with stirring under nitrogen and heated to reflux. A solution of 5, 5'-bis(bromomethyl)-2,2-bipyridyl (0.80g, 2.34mmol) in acetonitrile (300cm³) was added dropwise and the resultant solution heated at reflux for 48h. The mixture was then cooled to room temperature and the volume of the solvent reduced to 400cm³. The solid was collected by filtration, washed with acetonitrile (3 x 50cm³) and dried under vacuum. The solid was then dissolved in water (250cm³) with stirring and a saturated aqueous solution of ammonium hexafluorophophate added until no further precipitation occurred. The mixture was stirred for lh to ensure complete precipitation of the product. This was then collected by filtration, washed with water (3 x 20cm³) and dried under vacuum over silica gel to produce a white solid (1.61g, 88%), m.p.>300°C. M.S.(FAB) [m/z-PF₆-]⁺ 639.5, [m/z-2PF₆-]⁺ 493.5, [m/z-4.4'-bipyridyl-2PF₆-]⁺ 338.4,
¹H NMR (DMSO, 270MHz) δ: 6.01 (4H, s, bipyCH₂), 8.04 (4H, d, ³J=5.9Hz, 4,4'H_{3'}), 8.16 (2H, d, ³J=8.4Hz, bipyH₄), 8.46 (2H, d, ³J=8.4Hz, bipyH₃), 8.68 (4H, d, ³J=6.7Hz, 4,4'H₃), 8.89 (4H, d, ³J=5.9Hz, 4,4'H_{2'}), 8.97 (2H, s, bipyH₆), 9.42 (4H, d, ³J=6.7Hz, 4,4'H₂) ¹³C NMR (DMSO, 67.8MHz) δ: 60.25 (bipyCH), 120.79, 121.91, 125.88, 130.67, 137.93, 140.80, 145.46, 149.78, 150.86, 152.91 and 155.19 (aromatic C)
Analysis calculated for C₃₂N₂₆F₁₂N₆P₂;C:49.0%, H:3.3%, N:10.7%. Found C:48.7%, H:3.3%, N:10.4%.

### 5,5'-Bis(N'-methyl-4,4'-bipyridinium-N-methylene)-2,2'-bipyridyl tetra(hexafluorophosphate)

The white solid (0.30g. 0.38mmol) was dissolved in nitromethane (10cm³) with stirring. To this was added methyl iodide (10cm³) and the resultant solution heated to reflux for 24h. After cooling to room temperature the orange precipitate was collected by filtration and dried under vacuum. The solid was then dissolved in water (200cm³) with warming and a saturated aqueous solution of ammonium hexafluorophosphate added until no further precipitation occurred. The solid was collected by filtration, washed with water (2 x 20cm³) and dried under vacuum over silica gel to give the tetra-cationic receptor as a white solid (0.38g. 89%), m.p.>300°C.
M.S.(FAB) [m/z-PF₆-]⁺ 959(w), [m/z-2PF₆-]⁺ 814, [m/z-3PF₆-]⁺ 669.
I.R. 3120/3100cm⁻¹ (aromatic CH stretch),
1640/1600/1580cm⁻¹ (C=C/C=N ring stretch), 840cm⁻¹ (br, PF₆-)
¹H NMR (CD₃CN, 270MHz) δ: 4.40 (6H, s, 4,4'N+-CH₃), 5.93 (4H, s, bipyCH₂), 8.04 (2H, d, ³J=8.2Hz, bipyH₄), 8.36 (4H, d, ³J=6.6Hz, 4,4'H_{3'}), 8.42 (4H, d, ³J=6.8Hz, 4'4'H₃), 8.54 (2H, d, ³J=8.1Hz, bipyH₃), 8.83 (2H, s, bipyH₆), 8.84 (4H, d, ³J=6.6Hz, 4.4'H_{2'}), 9.02 (4H, d, ³J=7.0Hz, 4.4'H₂)
¹³C NMR (DMSO, 22.5MHz) δ: 48.02 (4,4'N⁺-CH₃), 60.80 (bipyCH₂), 120.88, 126.12, 127.06, 130.48, 138.08, 145.95, 146.57, 148.16, 149.33, 149.82 and 155.22 (aromatic C)
Analysis caluclated for C₃₄H₃₂F₂₄N₆P₄; C:37.0%, H:2.9%, N:7.6%. Found C:36.4%, H:2.7%, N:77.7%.

### N,N'-Dimethyl-5,5'-bis (N'-dimethyl-4,4'-bipyridinium-N-methylene)-2,2'bipyridyl hexa(hexafluorophosphate) (2)

The tetra-cationic receptor (100mg, 0.091mmol) was dissolved in acetonitrile (5cm³). To this was added dimethyl sulphate (5cm³) and the resultant solution heated at 80°C under nitrogen with stirring for 48h. After this time the solution was cooled to room temperature and dichloromethane (20cm³) added to precipitate the product as a yellow solid. This was collected by filtration and washed with dichloromethane (2 x 10cm³). The solid was then dissolved in water (50cm³) and a saturated aqueous solution of ammonium hexafluorophosphate added until precipitation ceased to occur. The product was collected by filtration, washed with water (2 x 10cm³) and dried under vacuum over silica gel to give a white solid (2) (92mg, 71%), m.p.>300°C.
M.S.(FAB) [m/z-2PF₆₋)⁺ 1134, [m/z-3PF₆₋]⁺ 989, [4,4'-CH₃]⁺ 171
I.R. 3150/3110cm⁻¹ (aromatic CH stretch).
1645/1605/1590cm⁻¹ (aliphatic CH stretch), 840cm⁻¹ (br, PF₆₋)
¹H NMR (DMSO, 400MHz) δ: 4.13 (6H, s, 4,4'N⁺-CH₃), 4.45 (6H, s, bipyN⁺-CH₃), 6.30 (4H, s, bipyCH₂), 8.47 (2H, d, ³J=8.1Hz, bipyH₄), 8.75 (4H, d, ³J=5.7Hz, 4,4'H_{3'}), 8.91 (4H, d, ³J=5.7Hz, 4,4'H₃), 9.07 (2H, d, ³J=7.9Hz, bipyH₃), 9.31 (4H, d, J=5.8Hz, 4,4'H_{2'}), 9.49 (4H, d, J=5.8Hz, 4,4'H₂), 9.67 (2H, s, bipyH₆) ¹³C NMR (DMSO, 100.6MHz) δ: 47.94 and 48.20 (bipyN⁺-CH₃ and 4,4'N⁺-CH₃), 59.21 (bipyCH₂), 126.17, 126.99, 130.75, 135.97, 142.87, 146.71, 146.88, 147.41, 147.77, 149.87 and 150.15 (aromatic C).
Analysis calculated for C₃₆H₃₈F₃₆N₆P₆; C:30.4%, H:2.7%, N:5.9%. Found C:24.8%, H:2.5%, N:5.9%.

### Anion Binding

The receptor (2) displayed substantial shifts of the signals of the ¹H NMR spectrum following the addition of one equivalent of tetrabutyl ammonium chloride. Analysis of the signals confirmed the formation of a complex between the receptor and the chloride ion.

### Example 3

### N,N'-Bis(5'-methyl-2,2'-bipyridyl-5-methylene)-4,4'-bipyridinium bis(hexafluorophosphate)

5-Bromomethyl-5'-methyl-2,2'bipyridyl (300mg, 1.14mmol) and 4,4'-bipyridyl (84mg, 0.54mmol) were dissolved in acetonitrile (20cm3) with stirring. The solution was then heated to reflux for 24h. After cooling to room temperature the precipitate was collected by filtration, washed with dichloromethane (2 x 10cm3) and dried under vacuum. It was then dissolved in water (75cm³) and a saturated aqueous solution of ammonium hexafluorophosphate added until precipitation ceased to occur. The solid wascollected by filtration, washed with water (2 x 10cm³) and dried under vacuum over silica gel to give a white powder. (343mg, 78%), m.p.>275°C (decomp).
M.S.(FAB) [m/z+H]⁺ 813, [m/z-PF₆⁻]⁺ 667, [m/z-2PF₆⁻]⁺ 522.
I.R. 3120/3090cm⁻¹ (aromatic CH stretch), 2920cm⁻¹ (aliphatic CH stretch), 1640/1600/1585cm⁻¹ (C=C/C=N ring stretch), 840cm⁻¹+ (br, PF₆₋)
¹H NMR (CD₃CN, 270MHz) δ: 2.39 (6H, s, bipyCH₃), 5.90 (4H, s, bipyCH₂), 7.73 (2H, d, ³J=8.1Hz, d, ⁴J=1.5Hz, bipyH_{4'}), 7.98 (2H, d, ³J=8.3Hz, d, ⁴J=2.2Hz, bipyH₄), 8.33 (2H, d, ³J=8.33Hz, bipyH_{3'}), 8.39 (4H, d,
³J=7.0Hz, 4,4'H₃), 8.48 (2H, d, ³J=8.2Hz, bipyH₃), 8.52 (2H, m, bipyH_{6'}), 8.78 (2H, d, ³J=1.8Hz, bipyH₆), 9.02 (4H, d, ³J=7.0Hz, 4.4'H₂)
¹³C NMR (DMSO, 22.5MHz) δ: 18.22 (bipyCH₃), 61.43 (bipyCH₂), 120.70, 127.66, 130.06, 134.68, 138.19, 138.32, 146.32, 149.80, 150.09, 152.34 and 156.69 (aromatic C).
Analysis calculated for C₃₄H₃₀F₁₂N₆P₂; C:50.3%, H:3.7%, N:10.3%. Found C:50.1%, H:3.4%, N:10.2%.

### N,N'-Bis(N,N',5'-trimethyl-2,2'-bipyridinium-5-methylene)-4,4'-bipyridinium bis(hexafluorophosphate) (3)

The white powder (200mg. 0.25mmol) was dissolved in acetonitrile (20cm³) under nitrogen. To this was added methyl iodide (10cm³) and the resultant solution heated at reflux for 6 days. After cooling to room temperature the solvent was removed under reduced pressure and the red solid dissolved in water (50cm³). To this was added a saturated aqueous solution of ammonium hexafluorophosphate until precipitation ceased to occur. The precipitate was collected by filtration, washed with water (2 x 10cm³) and dried under vacuum over silica gel.
The solid was then dissolved in acetronitrile (15cm³), methyl iodide (10cm³) added and the solution heated at reflux under nitrogen for 10 days. After cooling to room temperature the methyl iodide was removed under reduced pressure. Then a saturated solution of tetra-butyl ammonium chloride in acetonitrile was added dropwise until no further precipitation occurred. The solid was collected by filtration and washed with acetonitrile (2 x 10cm³). It was then dissolved in water (15cm³), a saturated aqueous solution of ammonium hexafluorophosphate added until no further precipitation occurred. The solid was collected by filtration, washed with water (2 x 10cm³) and then dried under vacuum over silica gel.
The resultant solid was dissolved in acetonitrile (10cm³), dimethyl sulphate (5cm³) added and the solution stirred at 75°C under nitrogen for 48h.
After cooling to room temperature the precipitate was collected by filtration. It was then washed with acetonitrile (3 x 20cm³). The solid was dissolved in water (20cm³) and a saturated aqueous solution of ammonium hexafluorophosphate added until no further precipitation occurred. The white solid was then collected by filtration, washed with water (3 x 10cm³) and dried under vacuum over silica gel to give a white powder.(3) (158mg, 44%).
M.S. (FAB) [m/z-2PF₆⁻]⁺ 1161
¹H NMR (DMSO, 400MHz) δ: 2.64 (6H, s, bipyCH₃), 4.05/4.16 (12H, 2s, bipyN⁺-CH₃), 6.29 (4H, s, bipyCH₂), 8.25 (2H, d, ³J=8.1Hz, bipyH_{4'}), 8.52 (2H, d, ³J=8.2Hz, bipyH₄), 8.74 (2H, d, ³J=8.1Hz, bipyH_{3'}), 8.89 (4H, d, ³J=6.4Hz, 4,4'H₃), 9.06 (2H, d, ³J=8.1Hz, bipyH₃), 9.36 (2H, s, bipyH_{6'}), 9.52 (4H, d, ³J=6.3Hz, 4,4'H₂), 9.67 (2H, s, bipyH₆)
¹³C NMR (DMSO, 100.6MHz) δ: 18.18 (bipyCH₃), 47.38 and 47.98 (bipyN⁺-CH₃), 59.42 (bipyCH₂). 127.15, 129.80, 131.03, 135.03, 135.62, 139.94, 141.69, 143.75, 146.72, 146.91, 147.40, 149.11, 149.54 and 150.25 (aromatic C).
Analysis calculated for C₃₈H₄₂F₃₆N₆P₆.2H₂O; C:30.7%, H:3.1%, N:5.6%. Found C:30.5%, H:2.9%, N:5.6%.

### Anion Binding

The receptor (3) displayed substantial shifts of the signals of the ¹H NMR spectrum following the addition of one equivalent of tetrabutyl ammonium chloride and tetrabutyl ammonium bromide, respectively. Analysis of the signals confirmed the formation of complexes between the receptor and the chloride ion and the bromide ion, respectively.

## Claims

1. An ion-sensitive compound having the formula Aⁿ⁺Bⁿ⁻ wherein A represents a cation capable of forming a receptor-substrate complex with an anion, B represents one or more counter anions and n is an integer from 3 to 10, characterised in that the cation is an anion receptor represented by the formula R¹-Y-R² wherein Y is represented by the structure 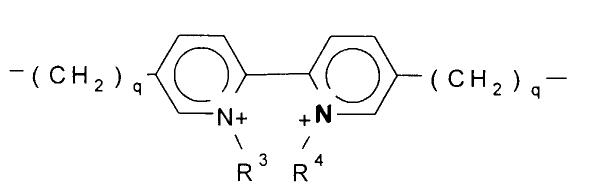
each q independently is an integer from 1 to 6;
R³ and R⁴ are each independently H or a lower alkyl group having from 1 to 4 carbon atoms, or R³ and R⁴ taken together represent an ethylene bridging group;
R¹ and R² are each independently selected from organic cations and non-ionic organic groups wherein at least one of R¹ and R² is an organic cation and the combined ionic charge of R¹ and R² is from 1 to 8 wherein
(a) the organic cation is selected from pyridinium, bipyridinium, alkylammonium, poly(alkylammonium), arylammonium and poly(arylammonium);
(b) the non-ionic organic group is selected from alkylamino, poly(alkylamino), arylamino, poly(arylamino), alkylamido, arylamido, alkylphosphoramido, arylphosphoramido, alkylsulphonamido, arylsulphonamido, alkyloxycarbonyl, aryloxycarbonyl, pyridyl and bipyridyl; or,
R¹ is H and R² is -X-Y-H wherein X is selected from alkylene, arylene, and quaternised or unquaternised amine- or polyamine-containing groups and Y is as defined above; or,
R¹ and R² taken together represent -X-Y-X- wherein X and Y are as defined above.

2. A compound according to claim 1 wherein each q represents 1.

3. A compound according to claim 1 or claim 2 wherein R³ and R⁴ each represent methyl.

4. A compound according to any one of the preceding claims wherein both R¹ and R² are organic cations.

5. A compound according to claim 4 wherein R¹ and R² are selected from 2,2'-bipyridinium, 4,4'-bipyridinium and [+N(R⁵)₂-R⁶]ₚ-R⁷ wherein each R⁵ independently is hydrogen or an alkyl group of from 1 to 4 carbon atoms; R⁶ is an alkylene group of from 1 to 3 carbon atoms; R⁷ is hydrogen or -N(R⁵)₃ and p is an integer from 1 to 4.

6. A compound according to any one of claims 1 to 3 wherein X is selected from alkylene, arylene, and quaternised or unquaternised amine- or polyamine-containing groups selected from alkylamino, arylamino, aminoalkyleneamino, amino[poly(alkyleneamino)], bipyridyl and bipyridylamino.

7. A compound according to claim 6 wherein X is 4,4'-bipyridinium or -[+N(R⁵)₂-R⁶]ₚ-N⁺(R⁵)₂- wherein each R⁵ independently is hydrogen or an alkyl group of from 1 to 4 carbon atoms; R⁶ is an alkylene group of from 1 to 3 carbon atoms; R⁷ is hydrogen or -N(R⁵)₃ and p is an integer from 1 to 4.

8. A compound according to claim 1 wherein the cation is N,N'-bis(N,N',5'-trimethyl-2,2'-bipyridyl-5-methylene)-N,N,N',N'-tetramethyl-ethylene-diammonium; N,N'-dimethyl-5,5'-bis(N'-dimethyl-4,4'-bipyridinium-N-methylene)-2,2'bipyridyl; or, N,N'-bis(N,N',5'-trimethyl-2,2'-bipyridinium-5-methylene)-4,4'-bipyridinium.

## Patentansprüche

1. Ionen-empfindliche Verbindung mit der Formel Aⁿ⁺Bⁿ, worin A für ein Kation steht, das mit einem Anion einen Rezeptor-Substrat-Komplex zu bilden vermag, worin B für ein oder mehrere Gegen-Anionen steht und n eine Zahl von 3 bis 10 ist, dadurch gekennzeichnet, daß das Kation ein Anionen-Rezeptor ist, der durch die Formel R¹-Y-R² dargestellt wird, worin Y durch die Struktur dargestellt wird: 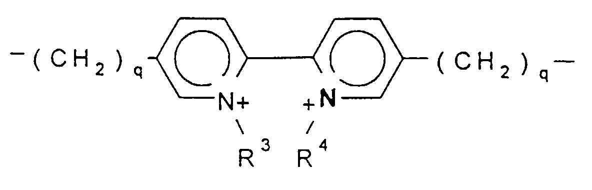 wobei
jedes q unabhängig voneinander für eine Zahl von 1 bis 6 steht;
R³ und R⁴ jeweils unabhängig voneinander stehen für H oder eine kurzkettige Alkylgruppe mit 1 bis 4 Kohlenstoffatomen, oder worin R³ und R⁴ gemeinsam eine eine Brücke bildende Ethylengruppe bilden;
R¹ und R² jeweils unabhäng voneinander ausgewählt sind aus organischen Kationen und nicht-ionogenen organischen Gruppen, worin mindestens eine der Gruppen R¹ und R² ein organisches Kation ist und wobei die kombinierte ionische Ladung von R¹ und R² bei 1 bis 8 liegt, worin
(a) das organische Kation ausgewählt ist aus Pyridinium, Bipyridinium, Alkylammonium, Poly(alkylammonium), Arylammonium und Poly(arylammonium);
(b) die nicht-ionogene organische Gruppe ausgewählt ist aus Alkylamino, Poly(alkylamino), Arylamino, Poly(arylamino), Alkylamido, Arylamido, Alkylphosphoramido, Arylphosphoramido, Alkylsulfonamido, Arylsulfonamido, Alkyloxycarbonyl, Aryloxycarbonyl, Pyridyl und Bipyridyl; oder worin
R¹ steht für H und R² steht für -X-Y-H, worin X ausgewählt ist aus Alkylen, Arylen und quaternisierten oder unquaternisierten Amin- oder Polyamin enthaltenden Gruppen, und worin Y die oben angegebene Bedeutung hat; oder worin
R¹ und R² gemeinsam stehen für -X-Y-X-, worin X und Y die oben angegebene Bedeutung haben.

2. Verbindung nach Anspruch 1, in der q jeweils steht für 1.

3. Verbindung nach Anspruch 1 oder Anspruch 2, worin R³ und R⁴ jeweils für Methyl stehen.

4. Verbindung nach einem der vorstehenden Ansprüche, worin R¹ und R² organische Kationen sind.

5. Verbindung nach Anspruch 4, worin R¹ und R² ausgewählt sind aus 2,2'-Bipyridinium, 4,4'-Bipyridinium und [⁺N(R⁵)₂-R⁶]ₚ-R⁷, worin R⁵ jeweils unabhängig voneinander steht für Wasserstoff oder eine Alkylgruppe mit 1 bis 4 Kohlenstoffatomen; R⁶ eine Alkylengruppe mit 1 bis 3 Kohlenstoffatomen ist; R⁷ für Wasserstoff oder -N(R⁵)₃ steht und p eine Zahl von 1 bis 4 ist.

6. Verbindung nach einem der Ansprüche 1 bis 3, worin X ausgewählt ist aus Alkylen, Arylen und quaternisierten oder unquaternisierten Amin- oder Polyamin enthaltenden Gruppen, ausgewählt aus Alkylamino, Arylamino, Aminoalkylenamino, Amino[poly(alkylenamino)], Bipyridyl und Bipyridylamino.

7. Verbindung nach Anspruch 6, in der X steht für 4,4'-Bipyridinium oder -[⁺N(R⁵)₂-R⁶]ₚ-N⁺(R⁵)₂- worin R⁵ jeweils unabhängig voneinander steht für Wasserstoff oder eine Alkylgruppe mit 1 bis 4 Kohlenstoffatomen; worin R⁶ steht für eine Alkylengruppe mit 1 bis 3 Kohlenstoffatomen; R⁷ steht für Wasserstoff oder -N(R⁵)₃ und worin p eine Zahl von 1 bis 4 ist.

8. Verbindung nach Anspruch 1, worin das Kation ist N,N'-Bis-(N,N',5'-trimethyl-2,2'-bipyridyl-5-methylen)-N,N,N',N'-tetramethyl-ethylen-diammonium; N,N'-Dimethyl-5,5'-bis(N'-dimethyl-4,4'-bipyridinium-N-methylen)-2,2'-bipyridyl; oder N,N'-Bis(N,N',5'-trimethyl-2,2'-bipyridinium-5-methylen)-4,4'-bipyridinium.

## Revendications

1. Composé sensible aux ions représenté par la formule Aⁿ⁺Bⁿ⁻, où A représente un cation capable de former un complexe récepteur-substrat avec un anion, B représente un ou plusieurs contre-ions et n est un entier de 3 à 10, caractérisé en ce que le cation est un récepteur d'anions représenté par la formule R¹-Y-R², où Y est représenté par la structure 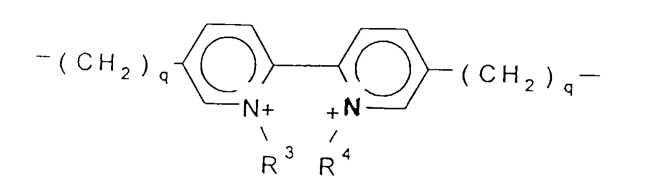
chaque q est indépendamment un entier de 1 à 6 ;
R³ et R⁴ sont chacun indépendamment H ou un groupe alkyle inférieur de 1 à 4 atomes de carbone ou R³ et R⁴, pris ensemble, représentent un groupe pontant éthylène ;
R¹ et R² sont chacun indépendamment choisis parmi les cations organiques et les groupes organiques non ioniques, où au moins l'un des groupes R¹ et R² est un cation organique et la charge ionique combinée de R¹ et de R² est de 1 à 8, dans lequel
(a) le cation organique est choisi parmi les groupes pyridinium, bipyridinium, alkylammonium, polyalkylammonium, arylammonium et polyarylammonium ;
(b) le groupe organique non ionique est choisi parmi les groupes alkylamino, polyalkylamino, arylamino, polyarylamino, alkylamido, arylamido, alkylphosphoramido, arylphosphoramido, alkylsulfonamido, arylsulfonamido, alkyloxycarbonyle, aryloxycarbonyle, pyridyle et bipyridyle ; ou
R¹ est H et R² est -X-Y-H dans lequel X est choisi parmi les groupes alkylène, arylène et les groupes contenant une amine ou une polyamine quaternisée ou non quaternisée et Y est tel que précédemment défini ; ou
R¹ et R², pris ensemble, représentent -X-Y-X-, dans lequel X et Y sont tels que précédemment définis.

2. Composé selon la revendication 1, dans lequel chaque q est 1.

3. Composé selon la revendication 1 ou 2, dans lequel R³ et R⁴ représentent chacun un groupe méthyle.

4. Composé selon l'une quelconque des revendications précédentes, dans lequel R¹ et R² sont des cations organiques.

5. Composé selon la revendication 4, dans lequel R¹ et R² sont choisis parmi les groupes 2,2'-bipyridinium, 4,4'-bipyridinium et [⁺N(R⁵)₂-R⁶]p-R⁷, où chaque groupe R⁵ est indépendamment l'hydrogène ou un groupe alkyle de 1 à 4 atomes de carbone ; R⁶ est un groupe alkylène de 1 à 3 atomes de carbone ; R⁷ est l'hydrogène ou un groupe -N(R⁵)₃ et p est un entier de 1 à 4.

6. Composé selon l'une quelconque des revendications 1 à 3, dans lequel X est choisi parmi les groupes alkylène, arylène et les groupes contenant une amine ou une polyamine quaternisée ou non quaternisée choisis parmi les groupes alkylamino, arylamino, aminoalkylèneamino, amino[poly(alkylèneamino)], bipyridyle et bipyridylamino.

7. Composé selon la revendication 6, dans lequel X est un groupe 4,4'-bipyridinium ou -[⁺N(R⁵)₂-R⁶]ₚ-N⁺(R⁵)₂⁻, où chaque groupe R⁵ est indépendamment l'hydrogène ou un groupe alkyle de 1 à 4 atomes de carbone ; R⁶ est un groupe alkylène de 1 à 3 atomes de carbone ; R⁷ est l'hydrogène ou un groupe -N(R⁵)₃ et p est un entier de 1 à 4.

8. Composé selon la revendication 1, dans lequel le cation est un groupe N,N'-bis(N,N',5'-triméthyl-2,2'-bipyridyl-5-méthylène)-N,N,N',N'-tétraméthyléthylène-diammonium ; N,N'-diméthyl-5,5'-bis(N'-diméthyl-4,4'-bipyridinium-N-méthylène)-2,2'-bipyridyle ou N,N'-bis(N,N',5'-triméthyl-2,2'-bipyridinium-5-méthylène)-4,4'-bipyridinium.
